Europäisches Patentamt

European Patent Office

Office européen des brevets

(11) Publication number: 0 341 477
A1

## EUROPEAN PATENT APPLICATION

(21) Application number: 89107335.5

(51) Int. Cl.⁴: C12N 15/00 , C12P 21/00

(22) Date of filing: 24.04.89

A request for correction of the description has been filed pursuant to Rule 88 EPC. A decision on the request will be taken during the proceedings before the Examining Division (Guidelines for Examination in the EPO, A-V, 2.2).

(30) Priority: 11.05.88 JP 113979/88

(43) Date of publication of application:
15.11.89 Bulletin 89/46

(84) Designated Contracting States:
DE FR GB

(71) Applicant: Chisso Corporation
6-32, Nakanoshima 3-chome Kita-ku
Osaka-shi Osaka-fu(JP)

(72) Inventor: Sakaki, Yoshiyuki
12-21, Takamiya 1-chome Minamiku
Fukuokashi Fukuokaken(JP)
Inventor: Ito, Takashi
7-21-303, Motoomachi
Nagasakishi Nagasakiken(JP)
Inventor: Tanahashi, Hiroshi
17-7, Higashi 4-chome Asakitaku
Hiroshimashi Hiroshimaken(JP)
Inventor: Inouye, Satoshi Haitsu Sumi 103
1-7, Teramae 1-chome Kanazawaku
Yokohamashi Kanagawaken(JP)

(74) Representative: Kraus, Walter, Dr. et al
Patentanwälte Kraus, Weisert & Partner
Thomas-Wimmer-Ring 15
D-8000 München 22(DE)

(54) A process for producing a photoprotein aequorin.

(57) A process for producing a jellyfish photoprotein aequorin in a mammalian cell system, the gene of which, when used as an expression gene marker, makes a high sensitivity detection possible by measuring a faint-luminescence activity in a non-radioisotope system, which process comprises cloning gene pAQ440 specifying the biosynthesis of a jellyfish photoprotein aequorin into an expression vector plasmid of a mammalian cell system, subjecting the resulting plasmid to transfection and producing the photoprotein aequorin in the mammalian cell.

## A process for producing a photoprotein aequorin

BACKGROUND OF THE INVENTION

1. Field of the Invention

This invention relates to a process for producing a jellyfish photoprotein aequorin in a mammalian cell system.

More particularly it relates to a process for producing a photoprotein aequorin which comprises cloning a gene specifying the biosynthesis of a jellyfish photoprotein aequorin into an expression vector of a mammalian cell system according to a recombinant DNA technique and using cells obtained by subjecting the resulting DNA to transfection to the mammalian cells.

2. Description of the Related Art

Calcium-binding photoprotein aequorin is existent around the periphery of the umbrella of jellyfish Aequorea growing in July to September near Friday Harbor Island in the suberbs of Seatle, Washington, U.S.A. and emits light by giving a stimulus thereto. As to the luminescent jellyfish, from 100 thousands thereof has been obtained about 200 mg of aequorin.

Aequorin is provided with properties that it combines specifically with calcium ion and emits light thus it has been utilized for determining calcium ion by detecting faint luminescence. In particular, it has been broadly utilized for measuring calcium ion in mammalian cells.

As described above, however, since the quantity of aequorin obtained from the natural luminescent jellyfish is so small that it cannot satisfy the demand in the market. Thus, the present inventors isolated an aequolin gene pAQ440 from the luminescent jellyfish according to recombinant DNA technique (Japanese patent application No. Sho 59-176,125/1984).

Further, we established the production system of apoaequorin in Escherichia coli as a prokaryotic cell (Japanese patent application No. Sho 61-249,098/1986), and also established the purification system thereof (Japanese patent application No. Sho 62-291,640/1987).

Due to these findings, the way for aequorin supply has been cleared; thus it is possible to expect the spreading effect thereof.

For example, when a sufficient quantity of purified aequorin is used, it is possible to elucidate the luminescent mechanism and principle thereof and also it is suggested that a possibility of its application research will be extended through cooperation of research in other fields.

Based on fundamental research up to date, the following properties of aequorin has been elucidated:

In general, the process of the development and differentiation of mammalian cells is presumed to be controlled mainly by the transcriptional level of gene, and the regulational signal of the gene expression has been analyzed by re-introducing a colored gene into the cells and observing the expression of the gene or the influence thereof upon the transcription.

Until now, various expression vectors have been developed in the mammalian cell systems, and as an expression gene marker, CAT gene (chloramphenicol acetyltransferase) has often been used. However, in the case of CAT assay method, the activity is determined by using $^{14}C$-chloramphenicol as a substrate and separating the resulting $^{14}$acetylchloramphenicol according to TLC.

It is indispensable for the CAT assay method to use a radioisotope, and at present, there has been desired an expression gene marker in place of the CAT gene, and having a higher safety at the time of its use, being of a non-isotope system and detecting with a high sensitivity.

SUMMARY OF THE INVENTION

The present inventors have made extensive research on such an expression gene marker, and have found that when calcium-binding photoprotein aequorin gene is used as the expression gene marker, a high sensitivity detection is possible by measuring a luminescent activity in a non-radioisotope system.

The present invention resides in a process for producing a photoprotein aequorin which comprises incorporating a gene pAQ440 specifying the biosynthesis of a jellyfish photoprotein aequorin into an

2

expression vector of a mammalian cell system, subjecting the resulting plasmid to transfection to the mammalian cell to introduce the gene and thereby produce apoaequorin and regenerating it into aequorin.

BRIEF DESCRIPTION OF THE DRAWINGS

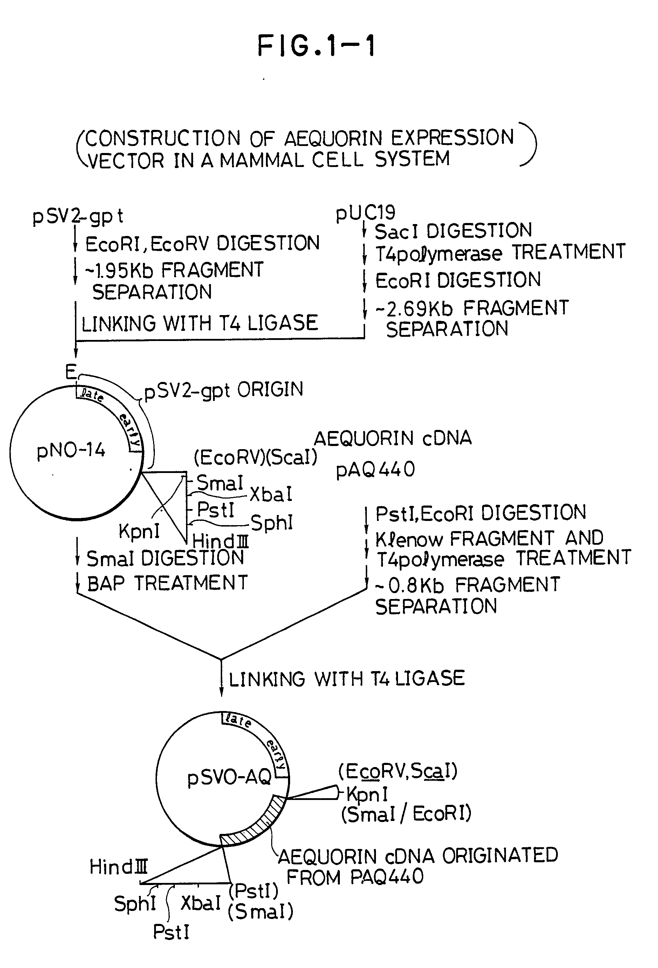

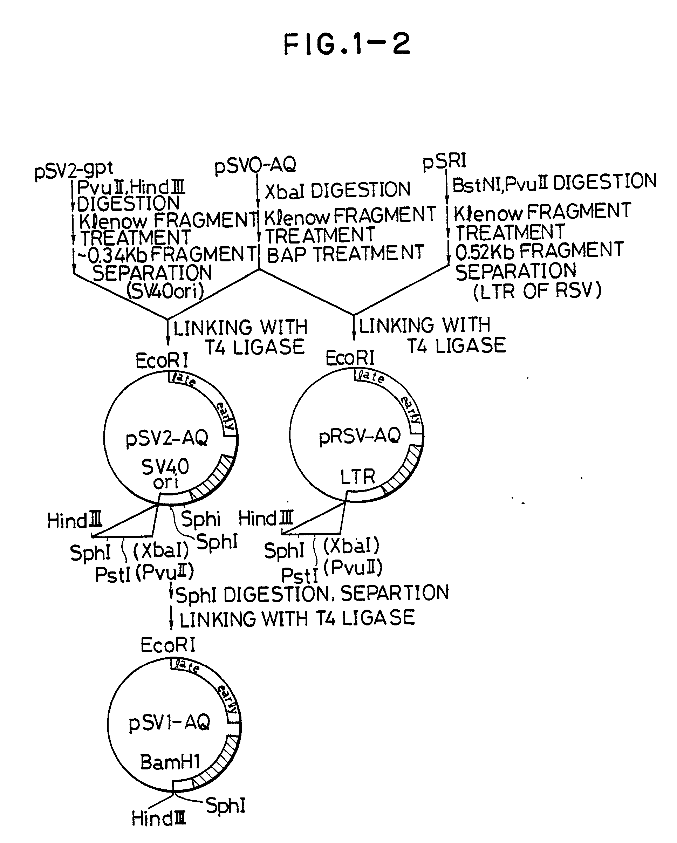

Fig. 1-1 and Fig. 1-2 each show a view illustrating an embodiment of the process of the present invention.

DETAILED DESCRIPTION OF PREFERRED EMBODIMENTS

As the mammalian cell system used in the present invention, for example a COS cell line-SV 40 system is usable. In the case of the gene expression in the COS cell line, in order that a vector plasmid DNA causes automatic growth in monkey kidney cells, it is necessary that $SV40_{ori}$ (replication-initiating point) be present in the vector plasmid DNA and SV40 large T (IT) antigen be present in the cells.

In the case of COS ($CV-1_{ori}$, SV40) cell line (Gluzman, Y. Cell, 23, 175 (1985)), since IT antigen is produced in monkey kidney CV-1 cells transformed by $SV40_{ori}$ defective variant, growth of SV40 initial variant is possible.

Thus, there were constructed pSV-AQ plasmid having aequorin gene exchanged with a portion of the gpt gene (gene of xanthine-guanine phosphoribosyltransferase) of a currently often used expression vector plasmid pSV2-gypt and the expression vector pRSV-AQ with LTR (Long terminal repeat) containing the expression promoter portion of retrovirus.

The present invention will be described in more detail by way of Example.

Example

① Construction of apoaequorin expression vector in a mammalian cell:

. Fig. 1 shows constructions of apoaequorin expression vectors, pSV2-AQ, pSVI-AQ and PRSV-AQ in a mammalian cell.

From an expression plasmid pSV-gpt (Mulligan R.C. et al, Proc. Natl. Acad. Sci., USA, 78, 2072 (1981), 5′, 3′ splicing region of SV40 DNA small T antigen mRNA and a portion containing a poly (A) additional signal for early mRNA, are separated after Eco RI/Eco RV digestion.

This fragment is subjected to subcloning at Sca I site and Eco RI site of cloning vector pUC19 (Yanisch. C. et al, Gene, 33, 110 (1985)) subjected to T4 polymerase treatment, to construct plasmid pNO-14.

The Pst I/Eco RI fragment of pAQ440 treated with Klenow fragment and T4 polymerase is inserted into the dephosphorylated Sma I site of pNO-14 to give pSVO-AQ. When the sequences of various promoters, enhancers, ori, etc. are inserted upstream of the aequorin gene of the pSVO-AQ, it is possible to easily construct an apoaequorin expression vector in a mammalian cell.

For example, ori region of SV40 (containing a promoter and an enhancer) was obtained from pSV2-gpt by Pvu II, Hind III digestion and was inserted into Xba I site of pSVO-AQ, to construct an apoaequorin expression-vector, pSV2-AQ, in COS cells.

pSV1-AQ formed from the pSV2-AQ by Sph I digestion and linking is possible to measure various enhancer activities using aequorin activity.

On the other hand, LTR (Long terminal repeat) of retrovirus contains a promoter which is controllable in mammalian cells. For example, a fragment containing LTR of RSV (Rous Sarcoma Virus) (Yamamoto T. et al, Proc. Natl . Acad Sci., U.S.A. (1981)) was inserted into Xba I site of pSV2-AQ to construct pRSV-AQ.

② Method of transfection of expression plasmid into mammalian cell:

(1) Mammalian cell used: COS cell originated from monkey kidney.
(2) Medium composition for animal cell cultivation:
The composition per 500 mℓ of medium is as follows:

| Dulbecco modified Eagle medium ② (made by Nippon Seiyaku Co., Ltd.) | 4.75 g |
| Fetal calf serum | 50 mℓ |
| L-glutamic acid | 0.292 g |
| 8.4% Sodium hydrogen carbonate | 9 mℓ |
| Penicillin G | 40,000 units |

(3) Transfection method:

COS cells are cultured using the above-mentioned medium, in a $CO_2$ incubater (5% $CO_2$, 37°C). COS cells at the logarithmic growth period thereof are successively planted in a 100 mm Falcon dish so as to give 1 $\times 10^6$ cells/10 mℓ, 24 hours before transfection. Further, the medium is exchanged with a fresh medium 4 hours before transfection.

Aequorin expression vector DNA (15 μg) is subjected to transfection to COS cells according to calcium phosphate method (F.L. Graham & A.J. Vander Eb, Virology, 52, 456 (1973)).
The composition is as follows:

| DNA 15μℓ | (1mM Tris-HCℓ (pH 7.5) 0.1 mM EDTA·2Na) |
| 219 μℓ | |
| 2M CaCℓ$_2$ | 31 μℓ |
| 2 × HBS | 250 μℓ (containing HEPES 10 g/ℓ, NaCℓ 16 g/ℓ, 70 mM Na$_2$HPO$_4$ and 70 mM NaH$_2$PO$_4$) |
| ~ 500 μℓ | |

After the transfection, culture was carried out as mentioned above for 12 hours, followed by exchanging the medium with a fresh medium and further carrying out cultivation for 36 hours under the same conditions.

③ Method of extracting the produced apoaequorin:

After the transfection for 48 hours, the medium in a Petri dish was removed, followed by three times washing the resulting cells with PBS (a solution of NaCℓ (8.0 g), KCℓ (0.2 g), Na$_2$HPO$_4$ (1.15 g) and KH$_2$PO$_4$ (0.2 g) dissolved in water (1 ℓ)) (a phosphate buffer solution conttaining neither $Ca^{2+}$ nor $Mg^{2+}$), further adding a small quantity of PBS, scraping off the cells on the Petri dish, collecting them into an Eppendorf tube, subjecting them to centrifugal separation by means of a table centrifuge at 5,000 r.p.m. for one minute to remove the resulting supernatant, suspending the collected cell precipitates in 30 mM Tris-HCℓ (pH 7.6) - 10 mM EDTA·2Na solution (200 μℓ), freezing the suspension with dry ice-methanol and three times repeating fusion at 37°C to break the cells, dyeing them with Trypan Blue to confirm the presence or absence of cell breakage, thereafter subjecting them to centrifugal separation at 14,000 r.p.m. at 4°C for 10 minutes and using the resulting supernatant as an extracting solution for the produced apoaequorin.

④ Method of converting apoaequorin into aequorin and method of measuring aequorin activity:

To the extracting solution for the produced apoaequorin are added 2-mercaptoethanol (6 μℓ) and coelenterazine (3 μℓ) (1 μg/μℓ solution), followed by making the total quantity 300 μℓ with 30 mM Tris-HCℓ (pH 7.6) - 10 mM EDTA·2Na solution and allowing the resulting material to stand on ice (4°C) for 12 hours to effect conversion into the generated aequorin.

The regenerated aequorin solution is placed in a 60 μℓ tube for measurement, followed by transferring it into a light-measuring chamber of Lumiphotometer TD-4000 made by Laboscience Company and injecting 30 mM CaCℓ$_2$/30 mM Tris-HCℓ (pH 7.6) solution (600 μℓ) therein to measure the initial maximum light intensity. The results are shown in Table 1.

## Table 1

| Expression plasmid | per $r\ell u/10^7$ cells |
|---|---|
| pSV1-AQ | 5 |
| pSV2-AQ | 520 |
| pRSV-AQ | - |

($r\ell u$: relative light units)

As apparent from the above results, the expression of aequorin gene in mammalian systems is possible and hence aequorin gene becomes a gene marker, and further, aequorin is produced in mammalian cells.

## Claims

1. A process for producing a photoprotein aequorin which comprises cloning a gene pAQ440 specifying the biosynthesis of a jellyfish photoprotein aequorin into an expression vector plasmid of a mammalian cell system, subjecting the resulting plasmid to transfection and producing the photoprotein aequorin in the mammalian cell after the transfection.

# FIG.1-1

(CONSTRUCTION OF AEQUORIN EXPRESSION
VECTOR IN A MAMMAL CELL SYSTEM)

EP 0 341 477 A1

# FIG.1-2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | EP-A-0 226 979 (CHISSO CORPORATION) * whole document * --- | 1 | C 12 N 15/00 C 12 P 21/00 |
| Y | EP-A-0 073 656 (GENENTECH. INC.) * abstract; page 5, line 23 - page 6, line 12; page 11, line 11 - page 13, line 6; page 20, line 11 - page 21, line 2; figures 1-3; claims 1,2,5-8 * --- | 1 | |
| A | CHEMICAL ABSTRACTS vol. 106, no. 19, 11 May 1987, abstract no. 150677x, Columbus, Ohio, US; M. NOGUCHI et al.: " Molecular cloning of apoaequorin cDNA"; & Methods Enzymol. 1986, vol. 133, pages 298-306 --- | 1 | |
| Y | EP-A-0 187 519 (UNIVERSITY OF GEORGIA RESEARCH FOUNDATION, INC.) * whole document * --- | 1 | |
| Y | CHEMICAL ABSTRACTS vol. 106, no. 25, 22 June 1987, abstract no. 208632x, Columbus, Ohio, USA; J. R. DE WET et al.: "Firefly luciferase gene: structure and expression in mammalian cells"; & Mol. Cell. Biol. 1987, vol. 7, no. 2, pages 725-737 --- | 1 | TECHNICAL FIELDS SEARCHED (Int. Cl.4) C 12 N 15/00 |
| A | EP-A-0 168 933 (SHIMON ULITZUR et al.) * abstract; page 6, lines 27-35; page 8, line 21 - page 9, line 19; page 12, line 36 - page 13, line 14 * --- -/- | 1 | |

The present search report has been drawn up for all claims

| Place of search | Date of completion of the search | Examiner |
|---|---|---|
| BERLIN | 02-08-1989 | JULIA P. |

EP 89 10 7335

# EUROPEAN SEARCH REPORT

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | BIOCHEMISTRY vol. 26, no. 5, 10 March 1987, pages 1326-1332, Washington, DC, USA; D. C. PRASHER et al.: " Sequence Comparisons of Complementary DNAs Encoding Aequorin Isotypes" * whole document; in particular page 1331, last paragraph * | 1 | |
| Y | CHEMICAL ABSTRACTS vol. 108, no. 8, 22 February 1988, abstract no. 70054t, Columbus, Ohio, USA; C. D. RASMUSSEN et al.: "Calmodulin is involved in regulation of cell proliferation"; & EMBO J. 1987, vol. 6, no. 13, pages 3961-3968 | 1 | |
| A | EP-A-0 191 606 (ELI LILLY AND COMPANY) * page 15, line 15 - page 16, line 16; page 18, line 9 - page 19, line 31; page 22, table I; page 41, example 2; claims 3-5 * | 1 | |
| T | CHEMICAL ABSTRACTS vol. 110, no. 23, 5 June 1989, abstract no. 207125e, Columbus, Ohio, USA; J. R. DE WET et al.: "Cloning and expression of the firefly luciferase gene in mammalian cells"; & Biolumin. Chemilumin., Proc. Int. Biolumin. Chemilumin. Symp., 4th 1986, (Pub. 1987), pages 369-372 | 1 | |

TECHNICAL FIELDS SEARCHED (Int. Cl.4)

The present search report has been drawn up for all claims

| Place of search | Date of completion of the search | Examiner |
|---|---|---|
| BERLIN | 02-08-1989 | JULIA P. |

EPO FORM 1503 03.82 (P0401)